(19) Europäisches Patentamt
European Patent Office
Office européen des brevets

(11) Veröffentlichungsnummer: **0 162 387**
**B1**

(12) # EUROPÄISCHE PATENTSCHRIFT

(45) Veröffentlichungstag der Patentschrift: **17.12.86**

(51) Int. Cl.⁴: **C 07 C 45/67**, C 07 C 49/00

(21) Anmeldenummer: **85105768.7**

(22) Anmeldetag: **10.05.85**

(54) **Verfahren zur Herstellung von Ketonen durch Isomerisierung von Aldehyden.**

(30) Priorität: **24.05.84 DE 3419378**

(43) Veröffentlichungstag der Anmeldung:
**27.11.85 Patentblatt 85/48**

(45) Bekanntmachung des Hinweises auf die Patenterteilung:
**17.12.86 Patentblatt 86/51**

(84) Benannte Vertragsstaaten:
**BE CH DE FR GB IT LI NL**

(56) Entgegenhaltungen:
**DE-A-2 758 113**
**US-A-3 466 334**
**US-A-4 329 506**

**SOVIET INVENTIONS ILLUSTRATED, Sektion Ch, Woche E 06, 24. März 1982, DERWENT PUBLICATIONS LTD., London, B 05**

(73) Patentinhaber: **BASF Aktiengesellschaft, Carl-Bosch- Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Merger, Franz, Dr., Max- Slevogt- Strasse 25, D-6710 Frankenthal (DE)**
Erfinder: **Mross, Wolf Dieter, Dr., Anselm-Feuerbach- Strasse 21, D-6710 Frankenthal (DE)**
Erfinder: **Fischer, Rolf, Dr., Bergstrasse 98, D-6900 Heidelberg (DE)**

**EP 0 162 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung von Ketonen durch Isomerisierung von Aldehyden unter Verwendung von Zeolithen als Katalysatoren.

Ketone sind wegen ihrer vielseitigen Verwendungsmöglichkeiten gesuchte chemische Verbindungen. Man verwendet sie z.B. als Lösungsmittel in der Gummi- und Kunststoffindustrie, als Lösungsmittel bei Chemischen Reaktionen, als Extraktionsmittel oder als Ausgangsstoffe für organische Reaktionen, z.B. als Zwischenprodukte für Farbstoffe, Pflanzenschutzmittel und Pharmaprodukte und als Duftstoffe. Die Herstellung von Ketonen aus Aldehyden durch Isomerisierung ist wünschenswert, da Aldehyde z.B. über die Oxosynthese leicht zugänglich sind. Isomerisierungen dieser Art sind bekannt. Man führt sie z.B. an Katalysatoren aus Zinn, Molybdän und Kupfer enthaltenden Mischoxiden (US-PS 4 329 506) oder an Ceroxid auf Aluminiumoxid (US-PS 3 466 334) durch. Von Nachteil bei diesen Verfahren ist, daß bei befriedigenden Umsätzen nur niedrige Selektivitäten erzielt werden und daß sich die besten Ergebnisse hinsichtlich Selektivität und Standzeit nur unter Zusatz von Wasserdampf erreichen lassen. Die schnelle Desaktivierung der Katalysatoren durch Koksabscheidung wirkt sich ebenfalls nachteilig aus. Man war deshalb bei der technischen Herstellung von unsymmetrisch substituierten Ketonen in der Regel auf die Kondensation unterschiedlicher organischer Säuren unter Decarboxylierung angewiesen, wie sie in der DE-OS 27 58 113 beschrieben ist. Bei diesem Verfahren ist der Zwangsanfall von symmetrisch substituierten Ketonen und von Kohlendioxid von Nachteil.

Es wurde nun gefunden, daß man bei der Herstellung von Ketonen durch Isomerisierung von Aldehyden an Katalysatoren besonders vorteilhafte Ergebnisse erzielt, wenn man die Isomerisierung bei Temperaturen bis 600° C unter Verwendung von Zeolithen als Katalysatoren vornimmt.

Nach dem neuen Verfahren werden hohe Selektivitäten, Umsätze und Standzeiten erzielt. Ein weiterer Vorteil ist, daß hohe Selektivitäten ohne Verlust der Katalysatoraktivität, d.h. bei langer Laufzeit auch unter Ausschluß von Wasserdampf erhalten werden.

Aldehyde, die sich nach dem erfindungsgemäßen Verfahren zu Ketonen isomerisieren lassen, sind z.B. Aldehyde der Formel

$$\begin{array}{l} \quad R^1 \\ \quad \vert \\ R^2\text{-}C\text{-}CHO \\ \quad \vert \\ \quad R^3 \end{array} \qquad I,$$

in der $R^1$ ein Wasserstoffatom oder einen Methyl- oder Ethylrest, $R^2$ ein Wasserstoffatom oder einen Alkylrest mit 1 bis 10 C-Atomen und $R^3$ einen Alkylrest mit 1 bis 10 C-Atomen, der noch Arylreste enthalten kann, einen Arylrest, einen Cyclopentyl- oder Cyclohexylrest oder einen heterocyclischen Rest bedeuten.

Die Alkylreste können linear oder verzweigt sein. Arylreste sind z.B. Phenylreste.

Als Aldehyde dieser Art seien z.B. Isobutyraldehyd, 2-Phenylpropanal, 2-Benzylpropanal, 2-Ethylhexanal, Pivalinaldehyd, 2-Ethylbutanal, 2-Methylbutanal und 2-Methylpentanal genannt. Die Aldehyde lassen sich z.B. durch Hydroformylierung von Olefinen herstellen. So läßt sich 2-Phenylpropanal aus Styrol durch Hydroformylierung gewinnen.

Als Katalysatoren werden für die erfindungsgemäße Isomerisierung von Ketonen zu Aldehyden Zeolithe eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions, ausgeglichen. Ein Kationenaustausch ist möglich. Die Räume zwischen den Tetraedern sind vor der Dehydratation durch Trocknen bzw. Calcinieren von Wassermolekylen besetzt. Die Zeolithe können auch anstelle des Aluminiums andere dreiwertige Elemente wie B, Ga, Fe oder Cr und anstelle des Siliciums andere vierwertige Elemente, wie Ge, enthalten.

Vorzugsweise werden als Katalysatoren Zeolithe des Pentasiltyps eingesetzt. Diese Zeolithe können unterschiedliche chemische Zusammensetzungen aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische. Besonders bevorzugt sind Alumino-, Boro- und Eisensilikatzeolithe des Pentasiltyps.

Der Aluminosilikatzeolith wird z.B. aus einer Aluminiumverbindung, vorzugsweise $Al(OH)_3$ oder $Al_2(SO_4)_3$, und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz bei 100 bis 220° C unter autogenem Druck hergestellt. Die erhaltenem Aluminosilikatzeolithe enthalten je nach Wahl der Einsatzstoffmengen ein $SiO_2/Al_2O_3$-Verhältnis von 10 bis 40.000. Die Aluminosilikatzeolithe lassen sich auch in etherischem Medium, wie Diethylenglykoldimethylether, in alkoholischem Medium, wie Methanol bzw. 1,4-Butandiol oder in Wasser herstellen.

Der Borosilikatzeolith wird z.B. bei 90 bis 200° C unter autogenem Druck synthetisiert, indem man eine

Borverbindung, z.B. $H_3BO_3$, mit einer Siliciumverbindung, vorzugsweise mit hochdispersem Siliciumdioxid, in wäßriger Aminlösung, insbesondere in 1,6-Hexandiamin- oder 1,3-Propandiamin-oder Triethylentetramin-Lösung mit und ohne Alkali- oder Erdalkalizusatz zur Reaktion bringt. Man kann bei dieser Reaktion anstelle einer wäßrigen Aminlösung eine etherische Aminlösung, z.B. mit Diethylenglykoldimethylether, oder eine alkoholische Lösung, z.B. mit 1,6-Hexandiol als Lösungsmittel verwenden.

Den Eisensilikatzeolith erhält man z.B. aus einer Eisenverbindung vorzugsweise $Fe_2(SO_4)_3$ und einer Siliciumverbindung, vorzugsweise aus hochdispersem Siliciumdioxid in wäßriger Aminlösung, insbesondere 1,6-Hexandiamin, mit und ohne Alkali- oder Erdalkalizusatz bei 100 bis 200°C unter autogenem Druck.

Die so hergestellten Alumino-, Boro- und Eisensilikatzeolithe können nach ihrer Isolierung, Trocknung bei 100 bis 160°C, vorzugsweise 110°C, und Calcination bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110°C/16 h getrocknet und bei 500°C/16 h calciniert. Besonders vorteilhaft lassen sich solche Katalysatoren dadurch herstellen, daß man den isolierten Alumino- bzw. Boro- bzw. Eisensilikatzeolith direkt nach der Trocknung verformt und erstmals nach der Verformung einer Calcination unterwirft. Aus dem zu Strängen verformten Katalysator kann man durch Mahlen und Sieben Wirbelgut mit der Teilchengröße vom 0,1 bis 0,5 mm erhalten. Die Alumino-, Boro- und Eisensilikatzeolithe können aber auch in reiner Form, ohne Binder, als Stränge oder Tabletten eingesetzt werden. Die Zeolithe können auch als Mordenit-Typ vorliegen.

Liegt der Zeolith aufgrund der Art seiner Herstellung nicht in der katalytisch bevorzugten aciden H-form, sondern z.B. in der Na-form vor, dann kann diese durch Ionenaustausch mit Ammoniumionen und anschließende Calcination oder durch Behandlung mit Säuren vollkommen oder partiell in die gewünschte H-Form übergeführt werden. Man kann an den Zeolithen zur Erhöhung der Selektivität und der Standzeit und der Anzahl an Regenerierungen auch unterschiedliche modifizierungen vornehmen. Eine geeignete modifizierung besteht z.B. darin, daß man den unverformten oder verformten Zeolithen mit Alkalin,tallen wie Na - sofern nicht schon von der Synthese her die Alkali-Form des Zeolithen vorliegt - mit Erdalkali wie Ca, Mg und Erdmetallen wie B, Tl ionenaustauschen bzw. imprägnieren kann. Insbesondere ist eine Dotierung der Zeolithe mit Übergangsmetallen, wie Mo, W, Fe, Zn, Cu, mit Edelmetallen, wie Pd und mit seltenen Erdmetallen, wie Ce, La, vorteilhaft.

Praktisch stellt man solche modifizierten Kontakte z.B. so her, daß man den verformten Pentasilzeolithen in einem Steigrohr vorlegt und bei 20 bis 100°C z.B. eine wäßrige Lösung eines Halogenids oder eines Nitrats der voranbeschriebenen Metalle darüberleitet. Ein derartiger Ionenaustausch kann z.B. an der Wasserstoff-, Ammonium- und Alkaliform des Zeolithen vorgenommen werden. Man kann die Metallaufbringung auf den Zeolithen z.B. auch so vornehmen, daß man das zeolithische Material z.B. mit einem Halogenid, einem nitrat oder einem Oxid der voranbeschriebenen Metalle in wäßriger oder alkoholischer Lösung imprägniert. Sowohl an einen Ionenaustausch als auch an eine Imprägnierung schließt sich zumindest eine Trocknung und wahlweise eine abermalige Calcination an.

Im einzelnen verfährt man z.B. so, daß man Molybdänoxid ($MoO_3$) oder Wolframsäure ($H_2WO_4$) oder $Ce(NO_3)_3x6H_2O$ in Wassergrößtenteils zumindest - löst. Mit dieser Lösung wird dann der verstrangte oder unverstrangte Zeolith eine gewisse Zeit, ca. 30 min., getränkt. Die überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcination bei ca. 500°C kann das so gewonnene zeolithische Material mit und ohne Bindemittel zu Strängen oder Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80°C in Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder mit Wasserdampf unterwirft. Dabei geht man vorteilhaft z.B. so vor, daß man das zeolithische Pulver vor seiner Verformung mit Flußsäure (0,001 n Bis 2 n, beforzugt 0,05 n bis 0,5 n) 1 bis 3 h unter Rückfluß behandelt. Nach Abfiltrieren und Auswaschen wird bei 100 bis 160°C getrocknet und bei 400 bis 550°C calciniert. Auch eine Behandlung der Zeolithe nach ihrer Verformung mit Bindemittel mit Salzsäure kann zweckmäßig sein. Hierbei wird der Zeolith z.B. 1 bis 3 h zwischen 60 und 80°C mit einer 3 bis 25 %igen, insbesondere mit einer 12 bis 20 %igen Salzsäure behandelt, anschließend ausgewaschen, bei 100 bis 160°C getrocknet und bei 400 bis 550°C calciniert. Man kann den Zeolithen auch durch Aufbringen von Phosphorverbindungen, wie Trimethoxyphosphat, modifizieren.

Nach einer Desaktivierung der zeolithischen Katalysatoren die beim Verfahren der Erfindung durch Koksabscheidung eintreten kann, lassen sich diese durch Abbrennen der Koksablagerung mit Luft oder mit

einem Luft/$N_2$-Ge-misch bei 400 bis 550° C, bevorzugt 500° C, in einfacher Weise regenerieren, wodurch sie ihre Anfangsaktivität zurückerhalten. Man kann auch durch eine partielle Verkokung (pre-coke) die Aktivität des Katalysators für ein Selektivitätsoptimum des gewünschten Reaktionsproduktes einstellen. Wird die Isomerisierung in Gegenwart von Gasen wie Wasserstoff, Stickstoff und Wasserdampf ausgeführt, so kann damit die Produktzusammensetzung und Standzeit des Katalysators beeinflußt werden. In allgemeinen werden die Katalysatoren wahlweise als 2 bis 4 mm Stränge, als Tabletten mit 3 bis 5 mm Durchmesser, als Pulver mit Teilchengrößen von 0,3 bis 0,5 mm oder (als Wirbelkontakt) von 0,1 bis 0,5 mm eingesetzt.

Die Isomerisierung der Aldehyde zu den Ketonen führt man an den Zeolithen vorzugsweise in der Gasphase bei Temperaturen von 100 bis 600° C, insbesondere bei 250 bis 500° C, durch. Die Belastung (WHSV) beträgt 0,1 bis 20, vorzugseise 0,5 bis 5 Gramm Aldehyd pro Gramm Katalysator und Stunde. Man kann auch in der Flüssigphase, z.B. bei Temperaturen von 30 bis 300° C isomerisieren. Das Verfahren kann chargenweise oder kontinuierlich, drucklos oder unter Druck, z.B. in einen Strömungsreaktor, Rührkessel oder Wirbelreaktor, durchgeführt werden. Unumgesetzte Aldehyde können gegebenenfalls nach der Umsetzung destillativ von den entstandenen Ketonen abgetrennt und erneut für die erfindungsgemäße Umsetzung verwendet werden.

**Beispiel 1**

2-Phenylpropanal wurde zum Zwecke der Isomerisierung zu Phenylaceton unter isothermen Bedingungen in einen Rohrreaktor (Wendelform, Innendurchmesser 0,6 cm und Länge von 90 cm) eingeführt und in der Gasphase bei Temperaturen von 400° C bzw. 350° C über einen Zeolith-Katalysator geleitet. Die erhaltenen Reaktionsprodukte wurden destillativ aufgearbeitet und durch Siedepunkt, Brechungsindex und NMR-Spektren charakterisiert. Die quantitative Bestimmung der Reaktionsprodukte und der Ausgangsprodukte erfolgte gaschromatographisch. Die Art des Katalysators, die Belastung (WHSV), der Umsatz und die Selektivität sind der folgenden Tabelle 1 zu entnehmen.

Tabelle I

| Katalysator | A | B | C | D | E | F | G | H | I | J |
|---|---|---|---|---|---|---|---|---|---|---|
| Temperatur °C | 400 | 400 | 350 | 400 | 400 | 400 | 400 | 400 | 400 | 400 |
| WHSV | 0,8 $h^{-1}$ | 2,4 $h^{-1}$ | 3,1 $h^{-1}$ | 2 $h^{-1}$ | 2,4 $h^{-1}$ | 2,4 $h^{-1}$ | 2,4 $h^{-1}$ | 2 $h^{-1}$ | 2,4 $h^{-1}$ | 2,4 $h^{-1}$ |
| Produktzusammensetzung Gew.% | | | | | | | | | | |
| Aldehyd | 37,0 | 53,6 | 32,9 | - | 21,9 | 46,2 | 20,0 | 16,1 | 33,2 | 8,5 |
| Keton | 61,3 | 42,3 | 64,5 | 86,6 | 73,0 | 49,8 | 77,1 | 77,3 | 56,3 | 78,2 |
| Umsatz % | 63,0 | 46,4 | 67,1 | 100 | 79,1 | 53,8 | 80,0 | 83,9 | 66,8 | 91,5 |
| Selektivität Keton % | 97,3 | 91,2 | 96,1 | 86,6 | 92,3 | 92,6 | 96,4 | 92,1 | 84,3 | 85,5 |

Die verwendeten Katalysatoren wurden folgendermaßen hergestellt:

**Katalysator A**

Der Katalysator wurde in einer hydrothermalen Synthese aus 64 g $SiO_2$ (hochdisperse Kieselsäure), 12,2 g $H_3BO_3$, 800 g einer wäßrigen Hexandiamin-Lösung (Mischung 50:50 Gew.%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/ 24 h calciniert. Es wurde ein Borosilikatzeolith vom Pentasiltyp erhalten, der 94,2 Gew.% $SiO_2$ und 2,32 Gew.% $B_2O_3$ enthielt. Aus diesem Zeolithen wurden 2 mm-Stränge hergestellt, die bei 100°C getrocknet und bei 500°C/24 h calciniert wurden.

**Katalysator B**

Ein Aluminosilikatzeolith von Pentasil-Typ wurde unter hydrothermalen Bedingungen bei autogenem Druck und 150°C aus 65 g hochdispersem $SiO_2$, 20,3 g $Al_2(SO_4)3$ x $18H_2O$ in 1 kg einer wäßrigen 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110°C/ 24 h getrocknet und bei 500°C/24 h calciniert. Dieser Aluminosilikatzeolith enthielt 91,6 Gew.% $SiO_2$ und 4,6 Gew.% $Al_2O_3$. Der Katalysator wurde zu 2 mm-Strängen verformt, bei 110°C/16 h getrocknet und bei 500°C/24 h calciniert.

**Katalysator C**

Der Eisensilikatzeolith des Pentasil-Typs wurde unter hydrothermalen Bedingungen bei autogenem Druck und 165°C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wurde abfiltriert, ausgewaschen, bei 100°C/24 h getrocknet und bei 500°C/ 24 h calciniert. Man erhielt einen Eisensilikatzeolithen mit einem $SiO_2$/ $Fe_2O_3$-Verhältnis von 17,7 und einen $Na_2O$-Gehalt von 1,2 Gew.%. Der Katalysator wurde zu 2,5 mm-Strängen verstrangt, bei 110°C/16 h getrocknet und bei 500°C/24 h calciniert.

**Katalysator D**

Dieser Katalysator wurde aus einem Eisensilikatzeolithen, wie bei Katalysator C beschrieben, durch Verformung zu Strängen mit Boehmit im Gewichtsverhältnis 60:40 und anschließende Calcination bei 500°C/16 h hergestellt Die Stränge wurden mit einer 20 %igen wäßrigen $NH_4Cl$-Lösung bei 80°C einem Ionenaustausch unterworfen und bei 500°C calciniert. Der Vorgang wurde mehrmals wiederholt, bis der Na-Gehalt des Katalysators unter 0,03 Gew.% abgefallen war.

**Katalysator E**

Der Katalysator wurde wie Katalysator B hergestellt, wobei jedoch das 1,6-Hexandiamin durch 1,3-Propandiamin ersetzt wurde. Der erhaltene Aluminosilikatzeolith enthielt 90,6 Gew.% $SiO_2$ und 3,4 Gew.% $Al_2O_3$.

**Katalysator F**

Der Katalysator wurde aus dem Aluminosilikatzeolithen, dessen Synthese bei Katalysator B beschrieben ist, hergestellt. Dabei wurde das Zeolithpulver mit hochdispersem $SiO_2$, das 0,3 bis 1,3 Gew.% hochdisperses $Al_2O_3$ enthielt, im Gewichtsverhältnis 60:40 zu 2 mm-Strängen verstrangt. Die Stränge wurden bei 110°C/16 h getrocknet und bei 500°C/16 h calciniert.

**Katalysator G**

Zur Herstellung des Katalysators wurde der gemäß A hergestellte Borosilikatzeolith mit HF behandelt. Hierbei wurden 50 g des Borosilikatzeolithen mit 140 ml 0,1 n HF 1 h unter Rückfluß gekocht. Nach Abfiltrieren wurde mit Wasser neutral gewaschen, bei 110°C/16 h getrocknet und bei 500°C/5 calciniert. Dieses Material wurde zu 5 mm Tabletten verpreßt.

**Katalysator H**

Der Katalysator wurde aus dem Katalysator G durch Verformung mit hochdispersem $SiO_2$, das 0,3 bis 1,3 Gew.% hochdisperses $Al_2O_3$ enthielt, im Gewichtsverhältnis 60:40 zu 2 mm-Strängen hergestellt.

**Katalysator I**

Katalysator A wurde mit Boehmit in Gewichtsverhältnis 60:40 zu 2 mm-Strängen verformt. Die bei 110°C/16 h getrockneten und bei 500°C/16 h calcinierten Stränge wurden mit einer wäßrigen Lösung von $Cu(NO_3)_2x3H_2O$ 30 min getränkt. Das Restwasser wurde am Rotationsverdampfer abgesaugt, die Stränge getrocknet und calciniert. Der Cu-Gehalt betrug 3,5 Gew.%.

**Katalysator J**

Es wurde wie unter Katalysator I beschrieben verfahren, wobei jedoch das $Cu(NO_3)_2x3H_2O$ durch $MoO_3$ ersetzt wurde. Der Tränkungsvorgang war - wenn nötig - zu wiederholen, bis die Stränge einen Mo-Gehalt von 1,1 Gew.% aufwiesen.

**Beispiel 2**

Zur Ermittlung des Einflusses der Temperatur auf den Umsatz und die Selektivität wurde Beispiel 1 bei Verwendung des Katalysators A bei 250°C und 500°C wiederholt. Die Ergebnisse sind aus Tabelle II ersichtlich.

**Tabelle II**

| Katalysator | A | A | A |
|---|---|---|---|
| Temperatur °C | 250 | 400 | 500 |
| WHSV | 0,8 $h^{-1}$ | 0,8 $h^{-1}$ | 0,8 $h^{-1}$ |
| Produktzusammensetzung Gew.% | | | |
| Aldehyd | 84,5 | 37,0 | 12,0 |
| Keton | 15,4 | 61,3 | 79,9 |
| Umsatz % | 15,5 | 63,0 | 88,0 |
| Selektivität Keton % | 99,3 | 97,3 | 90,8 |

**Beispiel 3**

Isobutyraldehyd wurde zum Zwecke der Isomerisierung zu Methylethylketon entsprechend der in Beispiel 1 beschriebenen Arbeitsweise umgesetzt. Die Ergebnisse sind aus der Tabelle III ersichtlich. TAbelle III>1

Tabelle III

| Katalysator | A | A |
|---|---|---|
| Temperatur °C | 400 | 500 |
| WHSV | 2 $h^{-1}$ | 2 $h^{-1}$ |

| | | |
|---|---|---|
| Umsatz % | 41,9 | 96,8 |
| Selektivität Keton % | 89,5 | 62,5 |

**Beispiel 4**

Pivalinaldehyd wurde zum Zwecke der Isomerisierung zu Methylisopropylketon entsprechend der in Beispiel 1 beschriebenen Arbeitsweise umgesetzt. Die Ergebnisse sind aus Tabelle IV ersichtlich.

| Katalysator | A | A |
|---|---|---|
| Temperatur °C | 250 | 350 |
| WHSV | 2 $h^{-1}$ | 2 $h^{-1}$ |

| | | |
|---|---|---|
| Umsatz % | 13,2 | 92,4 |
| Selektivität Keton % | 80,1 | 84,5 |

**Patentansprüche**

1. Verfahren zur Herstellung von Ketonen durch Isomerisierung von Aldehyden in Gegenwart von Katalysatoren, dadurch gekennzeichnet, daß man Aldehyde der Formel

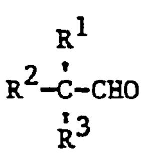

I,

in der $R^1$ ein Wasserstoffatom oder einen Methyl- oder Ethylrest, $R^2$ ein Wasserstoffatom oder einen Alkylrest mit 1 bis 10 C-Atomen und $R^3$ einen Alkylrest mit 1 bis 10 C-Atomen, der noch Arylreste enthalten kann, einen Arylrest, einen Cyclopentyl- oder Cyclohexylrest oder einen heterocyclischen Rest bedeuten, bei Temperaturen bis 600° C an einem Zeolithen als Katalysatoren umsetzt.

2. Verfahren nach Anspruch 1, dadurch gekennzeichnet, daß man 2-Phenyl-propanal zu Phenylaceton isomerisiert.

3. Verfahren nach Anspruch 1, dadurch gekennzeichnet, daß man Isobutyraldehyd zu Methylethylketon isomerisiert.

4. Verfahren nach Anspruch 1, dadurch gekennzeichnet, daß man Pivalinaldehyd zu Methylisopropylketon isomerisiert.

5. Verfahren nach Anspruch 1, dadurch gekennzeichnet, daß man Zeolithe vom Pentasil-Typ verwendet.

6. Verfahren nach Anspruch 1, dadurch gekennzeichnet, daß man einen Borosilikatzeolith des Pentasil-Typs verwendet.

7. Verfahren nach Anspruch 1, dadurch gekennzeichnet, daß man einen Aluminosilikatzeolith des Pentasil-Typs verwendet.

8. Verfahren nach Anspruch 1, dadurch gekennzeichnet, daß man einen Eisensilikatzeolith des Pentasil-Typs verwendet.

9. Verfahren nach Anspruch 1, dadurch gekennzeichnet, daß man die Umsetzung der Aldehyde an den Zeolithen in der Gasphase bei Temperaturen von 250 bis 500°C vornimmt.

## Claims

1. A process for the preparation of a ketone by isomerization of an aldehyde in the presence of a catalyst, wherein an aldehyde of the formula

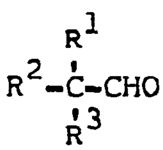 I,

where $R^1$ is hydrogen or methyl or ethyl, $R^2$ is hydrogen or alkyl of 1 to 10 carbon atoms, and $R^3$ is alkyl of l to 10 carbon atoms which may furthermore contain aryl, or is aryl, cyclopentyl, cyclohexyl or a heterocyclic radical, is converted at temperatures up to 600°C over a zeolite as the catalyst.

2. A process as claimed in claim 1, wherein 2-phenyl-propanal is isomerized to phenylacetone.

3. A process as claimed in claim 1, wherein isobutyraldehyde is isomerized to methyl ethyl ketone.

4. A process as claimed in claim 1, wherein pivalaldehyde is isomerized to methyl isopropyl ketone.

5. A process as claimed in claim 1, wherein a zeolite of the pentasil type is used.

6. A process as claimed in claim 1, wherein a borosilicate zeolite of the pentasil type is used.

7. A process as claimed in claim 1, wherein an aluminosilicate zeolite of the pentasil type is used.

8. A process as claimed in claim 1, wherein an iron silicate zeolite of the pentasil type is used.

9. A process as claimed in claim 1, wherein the conversion of the aldehyde over the zeolite is carried out in the gas phase at temperatures of from 250 to 500°C.

## Revendications

1. Procédé pour la préparation de cétones par isomérisation d'aldéhydes en présence de catalyseurs, caractérisé en ce que l'on fait réagir un aldéhyde répondant à la formule

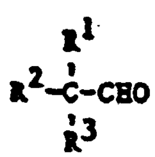 I,

dans laquelle $R^1$ est un atome d'hydrogène ou un radical méthyle ou éthyle, $R^2$ est un atome d'hydrogène ou un radical alkyle en $C_1$-$C_{10}$ et $R^3$ est un radical alkyle en $C_1$-$C_{10}$, qui peut contenir en outre un radical aryle, un radical cyclopentyle ou cyclohexyle ou un radical hétérocyclique, à des températures de 600°C sur une zéolithe comme catalyseur.

2. Procédé selon la revendication 1, caractérisé en ce que du 2-phényl-propanal est isomérisé en phénylacétone.

3. Procédé selon la revendication 1, caractérisé en ce que de l'isobutyraldéhyde est isomérisé en méthyléthylcétone.

4. Procédé selon la revendication 1, caractérisé en ce que de l'aldéhyde pivalique est isomérisé en méthylisopropylcétone.

5. Procédé selon la revendication 1, caractérisé en ce que l'on utilise une zéolithe du type pentasil.

6. Procédé selon la revendication 1, caractérisé en ce qu'on utilisé une zéolithe à base de borosilicate du type pentasil.

7. Procédé selon la revendication 1, caractérisé en ce que l'on utilise une zéolithe à base d'aluminosilicate du type pentasil.

8. Procédé selon la revendication 1, caractérisé en ce que l'on utilise une zéolithe à base de silicate de fer du type pentasil.

9. Procédé selon la revendication 1, caractérisé en ce que la réaction de l'aldéhyde sur la Zéolithe est effectuée en phase gazeuse à des températures de 250 à 500° C.